# EUROPÄISCHE PATENTSCHRIFT

(11) **EP 3 272 315 B1**
(45) Veröffentlichungstag und Bekanntmachung des Hinweises auf die Patenterteilung: **09.08.2023**
(21) Anmeldenummer: 17176296.6
(22) Anmeldetag: 16.06.2017
(51) Int. Cl.: A61F 2/44, A61F 2/46, A61F 2/30

(54) **INSTRUMENT ZUM EINBRINGEN EINES WIRBELSÄULEN-IMPLANTATS UND WIRBELSÄULEN-IMPLANTAT**
INSTRUMENT FOR INTRODUCING A SPINAL IMPLANT AND SPINAL IMPLANT
INSTRUMENT D'INTRODUCTION D'UN IMPLANT VERTÉBRAL ET IMPLANT VERTÉBRAL

(30) Priorität: 21.07.2016 DE 102016113488
(43) Veröffentlichungstag der Anmeldung: 24.01.2018
(73) Patentinhaber: MEDICON EG CHIRURGIEMECHANIKER-GENOSSENSCHAFT, 78532 Tuttlingen (DE)
(72) Erfinder: BÖHM, Heinrich, 99425 Weimar (DE); BURGER, Andreas, 78532 Tuttlingen (DE); WIDMAIER, Gerd, 78532 Tuttlingen (DE); WENZLER, Klaus, 78665 Frittlingen (DE)
(74) Vertreter: Mammel und Maser Patentanwälte PartG mbB

(56) Entgegenhaltungen:
- EP-A1- 2 424 469
- EP-B1- 2 424 469
- US-A1- 2007 093 897
- US-A1- 2008 221 694
- US-A1- 2012 271 422
- US-A1- 2013 103 102
- US-B1- 9 445 918

## Beschreibung

Die vorliegende Erfindung betrifft Bedieninstrumente und darauf adaptierte Wirbelsäulenimplantate, die von allen derzeit gebräuchlichen, konventionellen und weniger- bis minimal-invasiven Zugangsvarianten aus in den Zwischenwirbelraum eingebracht und z.B. bei "Tlif" oder "Plif" vor Ort mechanisch sicher geführt in die optimale Dauerposition gebracht werden können.

Bei fortgeschrittener Degeneration von Bandscheiben im Lendenwirbelsäulenbereich ist die Versteifungsoperation häufig die beste Behandlungsoption. Dazu werden die Reste der zerschlissenen Bandscheibe entfernt und Knochen- oder Knochenersatzmaterial in den Defekt eingebracht mit dem Ziel einer Fusion, also dem Zusammenwachsen der angrenzenden Wirbel zu einem Block. Dazu ist erstens wichtig, die für eine knöcherne Heilung erforderliche mechanische Stabilität zu gewährleisten und zweitens, die für die ungestörte Nervenfunktion nötigen geometrischen (=anatomischen) Lageverhältnisse wiederherzustellen. Dies zu gewährleisten, haben sich weltweit Platzhalter-Implantate, sogenannte "Cages", zur Rekonstruktion der vor dem Rückenmarkssack liegenden Wirbelkörpersäule durchgesetzt. Der direkte Implantationsweg dazu führt entsprechend durch den Bauchraum und wird unter Verwendung von konventionell offenen (anteriore lumbale intersomatische Fusion, "Alif") oder auch minimal-invasiven (z.B. "X-lif", "O-lif") operativen Zugangstechniken verwendet. Weil aber die meisten dieser Krankheitsbilder operative Maßnahmen auch an den dorsal des Spinalkanals liegenden Strukturen erfordern, stellt sich die Frage, ob der zusätzliche vordere Eingriff nicht vermieden werden kann und die rekonstruktiven Maßnahmen der vorderen Säule von dorsal, an Rückenmarkssack und Nervenwurzeln vorbei, erfolgen können. Die entsprechenden Operationsverfahren (posteriore lumbale intersomatische Fusion, "PLIF", und transforaminale intersomatische Fusion, "Tlif", sind etabliert und klinische Routine. Den engen anatomischen Platzverhältnissen folgend werden dabei kleinere Cages von dorsal, seitlich des Rückenmarkssackes in den ehemaligen Bandscheibenraum eingebracht. Dabei diktiert die Richtung des Zugangswegs die Dauerposition des Implantats. Unberücksichtigt oder schlecht rekonstruierbar bleibt bei den meisten dieser Cageversorgungen, dass lumbale Bandscheiben vorne höher sind als hinten, was ein normales Profil, "Lordose", bewirkt und dass ein möglichst großflächiges Implantat mit Position in der Nähe und parallel zur Vorderkante die optimale Lastüberleitung gewährleistet.

Die Implantate werden nach dem Ausräumen des Bandscheibenraumes von einem dorsalen Zugang her in den Raum zwischen den angrenzenden Wirbeln eingebracht und können dann in die Koronarebene gedreht bzw. geschwenkt werden. Dabei soll der konvexe Bereich des Cages parallel zur vorderen Begrenzung des Bandscheibenraumes positioniert werden.

Um das Implantat sicher in den Zwischenwirbelraum einzuführen, sollte das Implantat zunächst möglichst starr mit dem Einbringwerkzeug verbunden sein. Das Implantat ist zu diesem Zweck gelenkig an dem distalen Ende eines Bedieninstruments befestigt. Über einen Schraubmechanismus, der am proximalen Ende des Bedieninstruments vorgesehen ist, kann das Implantat in koaxialer Ausrichtung gegen das distale Ende des rohrförmig ausgebildeten Bedieninstruments gezogen und dort winkelstabil arretiert werden. Anschließend kann das Implantat unter dem Schutz von Dura und austretender Nervenwurzel in den Zwischenwirbelraum eingebracht, erforderlichenfalls auch eingeschlagen werden. Dabei bahnen Rillen an den Kontaktflächen zu den Nachbarwirbeln und die spezifisch ausgeformte Implantatspitze ("bullet nose") den Weg. Nach Passage der nervalen Strukturen wird das Bedieninstrument, soweit möglich, in der Transversalebene geschwenkt, die Verspannung im Gelenk durch Drehen des Griffs gelöst und das Instrument auf die andere Seite der Wunde durch Schwenken nachgeführt.

In dieser geänderten Stellung zum Bedieninstrument wird durch Spannen des Verschlusshebels die Gelenkverbindung zum Implantat wieder arretiert und das Bedieninstrument in die Ausgangsstellung zurückgedreht. Dieser Schwenkprozess wird so oft wiederholt, bis das Implantat in dem ehemaligen Bandscheibenraum von der Sagittalrichtung in die Koronarebene gedreht ist.

Dies ist beispielsweise in der DE 10 2013 005 692 A1 oder der EP 2 419 033 B1 beschrieben.

Aus der WO 2005/041825 A1 ist ein bogenförmiger intersomatischer Cage bekannt, der an seinen beiden Enden einen Durchbruch und eine in den Durchbruch mündende Nut aufweist, die zum Eingriff einer Stange mit einer Befestigungsnocke dient, wobei die Befestigungsnocke in einer Nut in dem Cage verschiebbar ist. Durch Betätigung eines Schraubmechanismus kann dann der Cage zusätzlich fest mit dem Bedieninstrument verschraubt werden, so dass eine starre Verbindung zwischen dem Cage und der Befestigungsvorrichtung entsteht und der Cage somit in den Zwischenwirbelraum eingeschlagen werden kann. Zur Lösung dieser starren Verbindung wird die Rändelschraube wieder gelöst, der Winkel zwischen der Längsachse des Cages und der Längsachse des Bedieninstruments verändert und dann kann der Cage weiter in den Zwischenwirbelraum eingebracht werden.

Aus der DE 10 2013 005 692 A1 ist ein Werkzeug mit zwei verschiedenen Einsätzen zum Einbringen von Wirbelsäulen-Implantaten bekannt. Das Bedieninstrument, ein Inserterstab, umfasst ein Außenrohr, das an seinem proximalen Ende einen hohlen Handgriff zur Aufnahme eines ersten Innenstabs aufweist. Der Innenstab ist mit einem über das distale Ende des Außenrohrs hinausreichenden Gewindebereich versehen. Der Gewindebereich an dem Innenstab dient zur Befestigung am Heckbereich des Implantats, das dort eine entsprechende Gewindebohrung aufweist. Über den gegenüber dem Außenrohr verschiebbaren Innenstab kann der Heckbereich des Implantats zudem gegenüber dem distalen Endbereich des Außenrohrs verspannt werden. Hierzu ist der distale Endbereich des Außenrohrs dem Heckbereich des Implantats zumindest teilweise angepasst. Die Arretierung erfolgt durch Betätigung eines Schraubgewindes. Beispiele von Bedieninstrumenten zur Implantierung eines Wirbelsäulen-Implantats sind aus der US 2008/221694 A1, aus der US 2013/103102 A1 und aus der US 2007/093897 A1 bekannt.

Um den Cage in den Bandscheibenraum einzubringen, bis sein hinteres Ende in Höhe des Anulus der Bandscheibe liegt, wird das zuvor beschriebene Inserter-Werkzeug eingesetzt, das dann, wenn die gewünschte Position erreicht ist, abgeschraubt und gegen ein spezielles "Ball"-Werkzeug ausgetauscht wird, um den Cage weiter einzutreiben und gleichzeitig ein Verdrehen/Verschwenken zu ermöglichen.

Das Erfordernis mehrerer Werkzeuge ist mühsam und erhöht die Gefahr für die Nervenstrukturen, die bei jedem Wechsel passiert werden müssen. Zudem wird in der Tiefe einer (blutenden) Wunde vorübergehend die Kontrolle über die aktuelle Implantatposition verloren und es lassen sich mit den bislang bekannten Werkzeugen die Implantate teilweise nicht in der exakt gewünschten Lage positionieren. Auch besteht bei manchen Implantaten die Gefahr, dass sich die Implantate während der Operation von dem Bedieninstrument ablösen. Wenn das Implantat einmal eingebracht und von dem Bedieninstrument gelöst wurde, ist es zudem schwer, es für eine Umpositionierung wieder an das Bedieninstrument anzudocken. Auch ist die Kontrolle über die Bewegung des Implantats schwierig. Zudem stört das Bedieninstrument eine Röntgenkontrolle. Sollte es erforderlich werden, auf eine andere Implantatgröße zu wechseln, so fehlt die geführte und sichere Möglichkeit der Entfernung.

Die Aufgabe der vorliegenden Erfindung besteht darin, ein Bedienwerkzeug und ein Wirbelsäulenimplantat anzugeben, die eine sichere, möglichst wenig traumatisierende und einfache Implantation ermöglichen.

Diese Aufgabe wird durch die Merkmale der Ansprüche 1 und 15 gelöst.

Erfindungsgemäß ist vorgesehen, dass das Implantat mit dem Außenrohr des Bedieninstruments über eine Spannvorrichtung mit einem Hebel verspannt wird, so dass ein einfaches und schnelles Lösen und Arretieren der Verspannung des distalen Endes des Heckteils des Implantats gegenüber dem distalen Ende des Außenrohrs ermöglicht wird.

Die Spannvorrichtung ist durch ein einfaches Betätigen eines Hebels bedienbar, so dass das zeitaufwendige und umständliche Schrauben und Lösen der bislang bekannten Schraubverbindungen vermieden wird.

Die erfindungsgemäße Spannvorrichtung weist eine Arretier- und eine Löseposition auf. Bei Betätigung des Hebels der Spannvorrichtung wird das Innenrohr in dem Außenrohr von einer gelösten Position in eine arretierte Position, in der der Heckbereich des Implantats mit dem distalen Endbereich des Außenrohrs verspannt ist, verschoben.

Vorzugsweise umfasst die Spannvorrichtung einen Hebel mit einem Exzenter.

Um eine besonders einfache und sichere Bedienung zu erreichen, ist die Spannvorrichtung vorzugsweise selbsthemmend, so dass Einrichtungen, die die Fixierung in der arretierten Position bewirken, entfallen können.

Besonders bevorzugt ist der Hebel an dem Handgriff oder einem gegenüber dem Handgriff verschiebbaren Rohrstück angeordnet. Der Hebel kann durch entsprechende Federelemente oder von Hand in die Löseposition zurückgestellt werden.

In einer bevorzugten Variante ist der Hebel der Spannvorrichtung auf einem in Längsrichtung verschiebbaren Rohrstück befestigt, in dem der Innenstab geführt ist, wobei das proximale Ende des Innenstabs auf dem proximalen Ende des Rohrstücks aufliegt. Durch die Betätigung des Hebels wird das Rohrstück und damit der auf dem Rohrstück aufliegende Innenstab gegenüber dem Handgriff in proximale Richtung verschoben, und das Implantat, beispielsweise durch den Exzenter, gegen das distale Ende des Innenstabs verspannt.

Die Verschiebung des Implantats in Richtung des distalen Endes des Außenrohrs und das Verspannen erfolgen somit nur durch Herunterdrücken des Hebels.

Auch kann am Rohrstück ein Federelement vorgesehen sein, das dazu dient, die Grundposition zu finden, in der das Implantat angebracht werden kann.

Die vorliegende Erfindung betrifft auch ein Bedieninstrument, bei dem der Innenstab hohl ist und bei dem ein weiterer Haltestab vorgesehen ist, der zur alleinigen Fixierung des Implantats während der Röntgenkontrolle dient, wobei der Haltestab in dem hohlen Innenstab aufnehmbar ist. Dadurch, dass der Innenstab hohl ist, kann in den hohlen Innenstab ein Haltestab hineingeschoben und mit dem Implantat vor der Röntgenkontrolle verschraubt werden. Anschließend wird das gesamte Bedieninstrument bis auf den mit dem Implantat verschraubten Haltestab entfernt und die Position des Implantats mit dem dünnen daran befestigten Haltestab durch Röntgen kontrolliert. Anschließend kann über den Haltestab das gesamte Bedieninstrument wieder auf den Haltestab aufgeschoben und das Bedieninstrument wieder an dem Implantat befestigt werden. Das Implantat kann somit nicht mehr im Zwischenwirbelraum verloren gehen.

Dieser Erfindungsaspekt ist unabhängig von der sonstigen speziellen Ausbildung des Bedieninstruments und insbesondere dem Vorhandensein einer Spannvorrichtung.

Der Haltestab weist in einer bevorzugten Ausführungsform an seinem distalen Ende einen Gewindebereich zur Befestigung am Implantat auf.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
Figur 1 die Draufsicht auf ein Implantat von oben,
Figur 2 eine Seitenansicht auf das Implantat aus Figur 1 auf die konkave Seite,
Figur 3 eine Seitenansicht auf das Implantat aus Figur 1 auf die konvexe Seite,
Figur 4 die Seitenansicht auf das Bedieninstrument mit hohlem Innenstab,
Figur 5 die Seitenansicht auf das Bedieninstrument ohne hohlen Innenstab,
Figur 6 die Seitenansicht auf das Bedieninstrument mit hohlem Innenstab und Haltestab,
Figur 7 eine Seitenansicht des hohlen Innenstabs,
Figur 8A eine Seitenansicht des Haltestabs,
Figur 8B eine vergrößerte Darstellung des Bereichs B aus Figur 8A,
Figur 9 eine Seitenansicht des Schraubschlüssels,
Figur 10 das Bedieninstrument mit Implantat in der Löseposition,
Figur 11 das Bedieninstrument mit dem Implantat aus Figur 10, jedoch in der Arretierposition,
Figur 12 eine Seitenansicht auf die Spannvorrichtung in Löseposition ohne Griff,
Figur 13 eine Seitenansicht auf die Spannvorrichtung in Arretierposition ohne Griff und
Figur 14 eine Detailansicht des Bereichs A in Figur 11, jedoch in einer anderen Winkellage des Implantats.

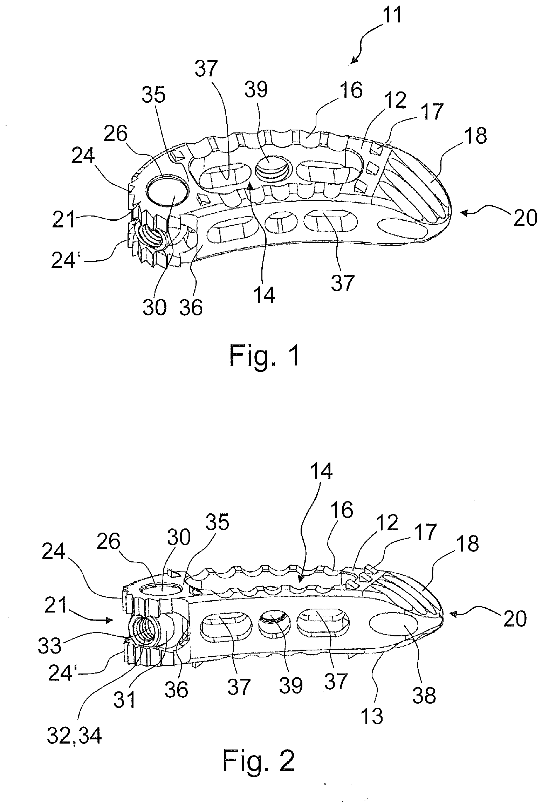

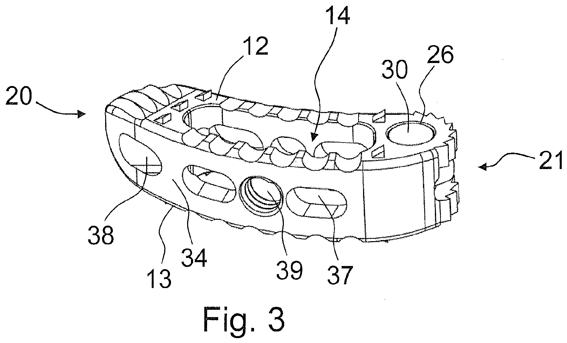

Das Implantat 11 in den **Figuren 1, 2** **und** **3** besitzt eine längliche, leicht gebogene bananenförmige Form mit einer Oberseite 12 und einer Unterseite 13. Durch die Ober- 12 und Unterseite 13 des Implantats 11 erstreckt sich eine vertikal verlaufende Durchgangsöffnung 14, die sich im mittigen Bereich des Implantats 11 erstreckt und der Aufnahme von Knochenmaterial und der besseren Durchknöcherung dient. Aufgrund des käfigartigen Aussehens werden solche Implantate 11 auch als "Cages" bezeichnet.

An der Ober- 12 und Unterseite 13 des Implantats 11 sind zudem Rillen 16 und Dorne 17, die der besseren Fixierung des Implantats 11 dienen. Die Rillen sind schräg angebracht, um bei Einschlagen des Implantates 11 dieses in einen Radius und letztendlich in die 90° Position zu bringen.

Die beiden seitlichen Enden 20, 21 des Implantats 11 sind unterschiedlich. An dem einen seitlichen Ende 20, dem Vorderende, ist das Implantat 11 angeschrägt und die Ober- 12 und Unterseite 13 verlaufen aufeinander zu. Dieses Ende 20 ist keilförmig ausgebildet ("bullet nose") und abgerundet, so dass das Implantat 11 mit diesem Ende 20 in den Zwischenwirbelraum eingeschlagen werden kann. Auf der Ober- 12 und Unterseite 13 des Vorderendes 20 verlaufen ebenfalls Rillen 18.

Das andere Ende des Implantats 11, das Implantatheck 21, ist von oben gesehen abgerundet und weist sowohl an der Ober- 12 als auch an der Unterseite 13jeweils einen Zahnkranz 24, 24' auf. Der Raum zwischen der Oberseite 12 und der Unterseite 13 am Implantatheck 21 ist im Wesentlichen offen. Zudem befinden sich in der Ober- 12 und Unterseite 13 zwei Bohrungen 26, die fluchten und in denen ein zylindrischer Zapfen 30 schwenkbar geführt ist. Von der Mantelfläche des Zapfens 30 erstreckt sich radial ein Gewindestück 31 in horizontale Richtung nach außen, das ein Innengewinde 32 mit einem größeren Durchmesser und ein Innengewinde 33 mit einem kleineren Durchmesser aufweist. Das Innengewinde 32 mit dem größeren Durchmesser dient zur Befestigung am distalen Ende 62 des Außenrohrs 61 und das Innengewinde 33 mit dem kleineren Durchmesser zur Befestigung des distalen Endes des Haltestabs 90, der - falls gewünscht - in das Außenrohr 61 hineingeschoben und mit seinem distalen Ende mit dem Innengewinde 33 mit dem kleineren Durchmesser verschraubt werden kann.

Das Innengewinde 33 mit dem kleineren Durchmesser erstreckt sich vom Ende des Innengewindes 32 mit größerem Durchmesser weiter in den Zapfen 30 hinein.

Der Zapfen 30 mit dem Gewindestück 31 kann in einem Winkel von wenigstens 50° und vorzugsweise wenigsten 60° in den Bohrungen 26 gegenüber dem Implantat 11 geschwenkt werden, weil das Implantat 11 in dem Schwenkbereich zwischen der Oberseite 12 und der Unterseite 13 im Wesentlichen offen ist. Seitlich ist die Schwenkbewegung des Zapfens 30 mit dem Gewindestück 31, die in einer Art Nut erfolgt, durch die Seitenwand 36 des Implantats 11 begrenzt, die einen Anschlag bildet.

Auf der in Richtung der Durchgangsöffnung 14 weisenden Seite des Zapfens 30 ist eine sich von der Ober- 12 zur Unterseite 13 des Implantats 11 erstreckende, durchgehende Wand 35, die dem Heckbereich 21 des Implantats 11 die erforderliche Stabilität verleiht.

In den seitlichen Außenwänden 34, 36 des Implantats 11, die die Ober- 12 und Unterseite 13 des Implantats 11 verbinden, sind ebenfalls Öffnungen 37, die der besseren Durchknöcherung dienen. Die konvexe Seitenwand 34 ist wegen der gewünschten Lordoseform geringfügig höher als die konkave Seitenwand 36.

Ebenso sind horizontal verlaufende Öffnungen 38, die sich von dem keilförmigen Vorderende 20 (bullet nose) in Richtung der vertikal verlaufenden Durchgangsöffnung 14 des Implantats 11 erstrecken, vorgesehen, die ebenfalls der besseren Durchknöcherung dienen.

In der konvexen Seitenwand 34 des Implantats 11 ist eine Aufnahmeöffnung 39 vorgesehen, die ein Innengewinde aufweist. Dies ermöglicht, nachdem das Bedieninstrument 60 oder ein weiteres konventionelles Einbringinstrument in die Aufnahmeöffnung 39 geschraubt wurde, auch ein Einbringen des Implantats 11 aus ventraler Richtung. Nach erfolgter Implantation dient die Aufnahmeöffnung 39 ebenfalls der besseren Durchknöcherung.

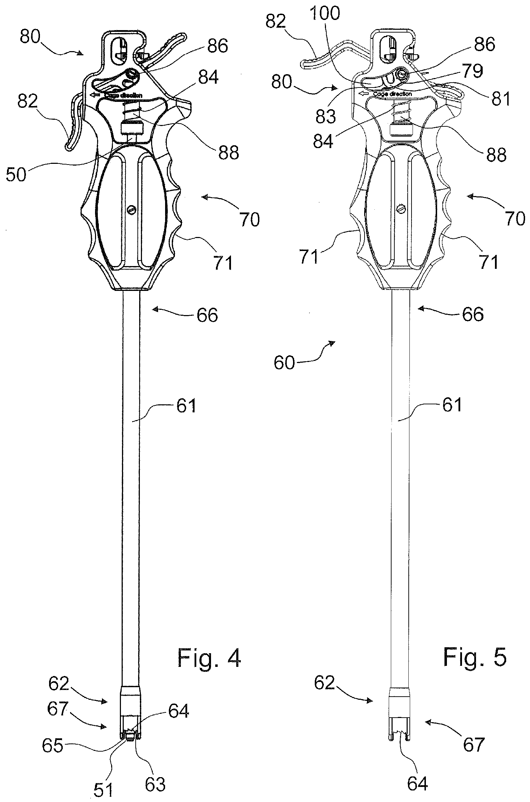

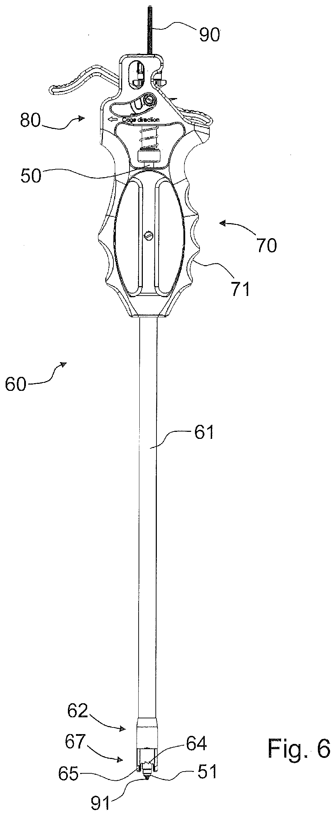

Das Bedieninstrument 60, das in den **Figuren 4bis 6** dargestellt ist, umfasst ein langes Außenrohr 61, das an seinem distalen Ende 62 eine zu der abgerundeten Form des Implantathecks 21 korrespondierende Ausnehmung 63 aufweist mit einem zu dem Zahnkranz 24, 24' am Heckbereich 21 des Implantats 11 korrespondierenden Zahnkranz 64. Somit ist der distale Endbereich 67 des Außenrohrs 61 dem Heckbereich 21 des Implantats 11 zumindest teilweise angepasst. Die Zähne der Zahnkränze 24, 24` am Implantat 11 und am Zahnkranz 64 des Bedieninstruments 60 greifen ineinander, um eine möglichst stabile Verbindung zu erzielen. Um einerseits die Drehbewegung des Implantats 11 gegenüber dem Bedieninstrument 60 während des Schwenkens zu erleichtern und um andererseits eine mögliche Rückwärtsbewegung des Implantats 11 beim Rotationsvorgang in die Koronarebene zu vermeiden, sind die Zähne der Zahnkränze 24, 24', 64 asymmetrisch. Zudem sind an dem distalen Ende 62 des Außenrohres 61 noch zwei sich in Längsrichtung erstreckende Stifte 65 vorgesehen, die hauptsächlich dazu dienen, im gelösten Zustand die Ausrichtung nicht zu verlieren, damit beim Wiederanziehen sich die Zähne wieder finden und darüber hinaus dazu dienen, das Gewindestück 31 seitlich zu umgreifen.

Am proximalen Ende 66 geht das Außenrohr 61 in den Handgriff 70 über, der mehrere Greifmulden 71 aufweist, so dass er mit der Hand vollständig umgriffen werden kann und gut in der Hand liegt. Im proximalen Endbereich des Handgriffs 70 befindet sich die Spannvorrichtung 80 zur Verspannung des Innenstabs 50.

Der Innenstab 50 ist in dem Außenrohr 61 verschieblich geführt. Der Innenstab 50 ist in dieser Ausführungsform als hohler Innenstab 50, d.h. als Innenrohr, ausgebildet. Das Innenrohr 50 ist an seinem distalen Ende mit einem über das distale Ende 62 des Außenrohrs 61 hinausreichenden Außengewinde 51 versehen, das mit dem Innengewinde 32 mit größerem Durchmesser in dem Gewindestück 31 des Zapfens 30 in dem Implantat 11 verschraubt werden kann.

Bei erfolgter Verschraubung ist das Implantat 11 unverlierbar an dem Innenrohr 50 befestigt, kann gegenüber diesem jedoch infolge des schwenkbaren Zapfens 30 geschwenkt werden.

Durch Betätigung des Spannmechanismus 80 kann der Zahnkranz 64 an dem distalen Ende 62 des Außenrohrs 61 mit dem Zahnkranz 24, 24' im Heckbereich 21 des Implantats 11 in Eingriff gebracht werden, zudem umgreifen die beiden Stifte 65 das Gewindeteil 31 an dem schwenkbaren Zapfen 30 auf der rechten und linken Seite, so dass das Implantatheck 21 mit dem Zahnkranz 24, 24' mit dem Außenrohr 61 starr verbunden ist. Zudem ist das Außenrohr 61 über die Stifte 65 auch starr mit dem Gewindeteil 31 und dem Zapfen 30 verbunden. Da das Innenrohr 50 über die Verschraubung 51, 32 auch starr mit dem Gewindeteil 31 des Zapfens 30 verbunden ist, kann auch der Zapfen 30 nicht mehr hin- und hergeschwenkt werden.

In dieser Arretierposition, die der gespannten Spannvorrichtung 80 entspricht und in der die Schraubgewinde 51, 32, Zahnkränze 24, 24', 64 und Stifte 65 alle in Eingriff gebracht sind, ist das Implantat 11 starr und sicher an dem Bedieninstrument 60 befestigt.

Somit wird im arretierten Zustand eine starre Verbindung zwischen Implantat 11 und Bedieninstrument 60 erreicht, und das Implantat 11 kann sicher geführt in den ausgeräumten Zwischenwirbelraum eingebracht werden.

Sobald das Implantat 11 die für die erforderliche Schwenkbewegung erforderliche Position erreicht und sich zwischen den Nachbarwirbeln verklemmt hat, kann die starre Verbindung zwischen den Zahnkränzen 24, 24', 64 durch Lösen des Spannmechanismus 80 aufgehoben und die das Gewindeteil 31 von außen umgreifenden Stifte 65 von dem Gewindeteil 31 wieder entfernt werden. In dieser "Löseposition" ist das Implantat 11 nur noch über die Verschraubung 32, 51 an dem Zapfen 30 mit dem Außenrohr 61 des Bedieninstruments 60 befestigt, und infolge der Schwenkbarkeit des Implantats 11 gegenüber dem Zapfen 30 ist somit nun auch eine Schwenkbewegung des Bedieninstruments 60 gegenüber dem Implantat 11 möglich.

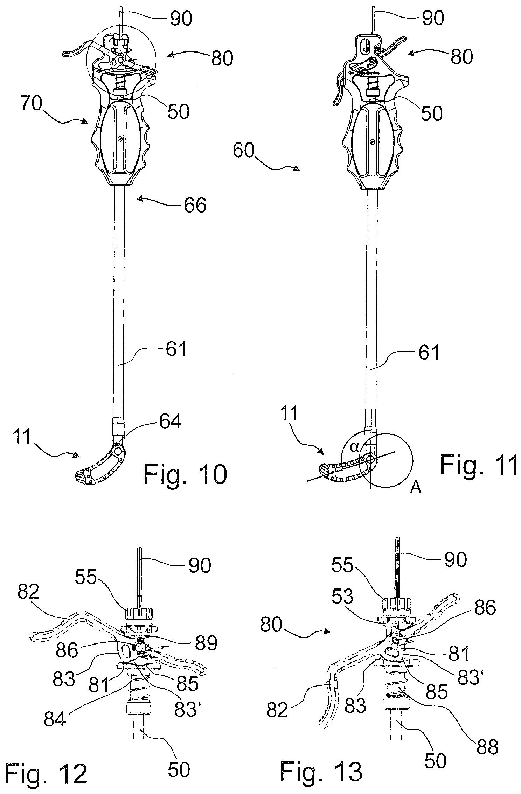

Somit kann nun durch Lösen der Spannvorrichtung 80 und damit wieder Herstellung der Bewegbarkeit das Bedieninstruments 60 gegenüber dem im Zwischenwirbelraum festsitzenden Implantat 11 der Winkel a zwischen der Längsachse des Außenrohrs 61 (vgl. Figur 11) und der Längsachse des Implantats 11 etwas verringert werden. Nun wird das Implantat 11 durch Spannen der Spannvorrichtung 80 wieder starr mit dem Bedieninstrument 60 verbunden, das Bedieninstrument 60 wird nun in der Operationsöffnung wieder geschwenkt bzw. gehebelt, bis es den Rand der Operationsöffnung erreicht hat. Die Spannvorrichtung 80 wird wieder geöffnet und der Winkel a wiederum verkleinert, das Implantat 11 wieder festgespannt und wieder ein weiteres Stück in der Transversalebene gedreht, bis das Implantat 11 nach mehrfacher Wiederholung dieses Vorgangs in die Endposition parallel zur Vorderkante geschwenkt ist. Die richtige Positionierung wird durch eine intraoperative Röntgenaufnahme kontrolliert. Hierfür ist das Bedieninstrument 60 so gestaltet, dass vermieden wird, dass die Implantatposition währenddessen verlorengeht oder dass das aus dem Körper ragende Bedieninstrument 60 während des Röntgens versehentlich in den Rückenmarkskanal verschoben wird und/oder eine höhere Strahlendosis triggert. Hierzu wird zunächst durch den Innenstab 50, der für diesen Zweck hohl als Innenrohr 50 ausgebildet ist, ein Haltestab 90 eingeführt, an dessen distalen Ende sich ein Außengewinde 91 mit einem kleinen Durchmesser befindet. Das Außengewinde 91 des Haltestabs 90 kann mit dem Innengewinde 33 mit dem kleineren Durchmesser in dem Gewindeteil 31 verschraubt werden, so dass das Implantat 11 an dem Haltestab 90 befestigt werden kann. Anschließend kann das gesamte Bedieninstrument 60 (d.h. mit Außenrohr 61 und mit Innenrohr 50) von dem Implantat 11 entfernt werden, bis auf den Haltestab 90, der an dem Implantat 11 über das Innengewinde 33 in dem Gewindeteil 31 befestigt bleibt, so dass die Position des Implantats 11 während des Röntgens nicht verloren gehen kann. Der Durchmesser des Haltestabs 90 ist gering und beträgt nur etwa 1 bis 2 mm, so dass die Röntgenaufnahme nur unwesentlich gestört wird.

Ergibt die Röntgenaufnahme, dass das Implantat 11 noch weiter eingebracht werden oder die Lage des Implantats verändert werden muss, so wird das Bedieninstrument 60 mit dem Innenrohr 50 und dem das Innenrohr 50 umgreifenden Außenrohr 61 wieder über das proximale Ende des Haltestabs 90 gestreift und das Innenrohr 50 wieder mit seinem distalen Ende 51 an dem Innengewinde 32 des Gewindeteils 31 des Implantats 11 befestigt.

Bei gelöster Spannvorrichtung 80 kann nun der Winkel zwischen Implantat 11 und Bedieninstrument 60 wieder verändert, die Spannvorrichtung 80 wieder gespannt werden etc. und das Implantat 11 so in die nächste gewünschte Position gebracht werden.

Am proximalen Ende 66 des Bedieninstruments 60 ist die Spannvorrichtung 80, mittels der in der einfachsten Variante das an dem Innenrohr 50 befestigte Implantat 11 mit seinem Zahnkranz 24, 24' gegen den sich am distalen Ende 62 des Außenrohrs 61 befindlichen Zahnkranz 64 gepresst und so in Eingriff gebracht werden kann.

Die Spannvorrichtung 80 ist eine Klemmvorrichtung, die sich durch Betätigung eines Hebels 82 lösen und festsetzen lässt und vorzugsweise in eine Selbsthemmung übergeht.

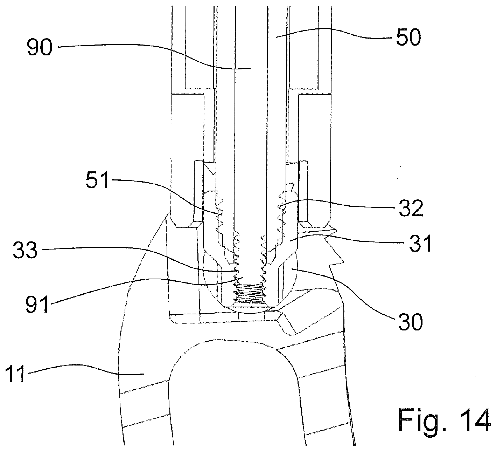

In den bevorzugten in den Figuren 4 bis 14 dargestellten Varianten ist die Spannvorrichtung 80 mit einem zweiseitigen Hebel 82 betätigbar, der etwa mittig drehbar an einer Drehachse 86 befestigt ist und von der Löseposition (Figur 10, Figur 12) in die Arretierposition (Figur 11, Figur 13) geschwenkt und dann wieder gelöst werden kann.

An der Unterseite des Hebels 82 ist ein Exzenter 81, der einstückig mit dem Hebel 82 verbunden ist. Der Exzenter 81 liegt mit seiner Unterseite 83 auf einer Gleitfläche 85 des Handgriffteils 70 auf. Bei Betätigung des Hebels 82 kommt es nun durch die in Bezug auf die Drehachse 86 unter zunehmend beabstandeten Spannflächen 83, 83' des Exzenters 81, die entlang der Gleitfläche 85 des Handgriffs 70 gleiten, zu einer zunehmenden Verspannung, die infolge von Reibung in Selbsthemmung übergeht.

Die Drehachse 86 und der Hebel 82 sind in der bevorzugten Variante auf einem verschiebbaren Rohrstück 88 befestigt, so dass bei einer Betätigung des Hebels 82 (Spannen) durch den Exzenter 81 das gesamte verschiebbare Rohrstück 88 einschließlich dem Hebel 82 in proximale Richtung verschoben und festgesetzt wird. Nachdem jedoch am proximalen Ende des verschiebbaren Rohrstücks 88 das Innenrohr 50 in das verschiebbare Rohrstück 88 hineingesteckt ist und das Innenrohr 50 einen Anschlag 53 aufweist, der sich an der Außenkante 89 des Rohrstücks 88 abstützt, wird bei Betätigung des Hebels 82 nicht nur das verschiebbare Rohrstück 88, sondern auch das Innenrohr 50 in proximale Richtung geschoben und dadurch das mit dem distalen Ende 51 des Innenrohrs 50 verschraubte Implantat 11 gegen das distale Ende 62 des Außenrohrs 61 und den dortigen Zahnkranz 64 gepresst und mit diesem in Eingriff gebracht, so dass in Arretierposition die Winkellage des Implantats 11 zum Außenrohr 61 fixiert ist.

Durch die Betätigung des Hebels 82 der Spannvorrichtung 80 kann somit schnell und auf einfache Weise eine starre Befestigung zwischen Implantat 11 und Bedieninstrument 60 bereitgestellt werden, die durch Lösen der Spannvorrichtung 80, d.h. Betätigung der anderen Seite des Hebels 82, einfach und schnell wieder gelöst werden kann.

Um das Innenrohr 50 nach Lösen der Spannvorrichtung 80 wieder in die Ausgangsposition rückzustellen, ist die Spannvorrichtung 80 auf dem verschiebbaren Rohrstück 88 befestigt, das in distaler Richtung einen Anschlag für eine die Mantelfläche des Rohrstücks 88 umgreifende Feder 84 aufweist. Auf der anderen Seite stützt sich die Feder 84 an dem Handgriff 70 ab. Wird die Spannvorrichtung 80 gelöst, so bewirkt die Feder 84 auf dem verschiebbaren Rohrstück 88, dass das Rohrstück 88 und damit das Innenrohr 50 in distale Richtung verschoben und damit in Löseposition gebracht wird.

Die an dem Rohrstück 88 vorgesehene Feder 84 stellt somit das Außenrohr 61 in der Löseposition wieder außer Eingriff mit dem Heckbereich 21 des Implantats 11.

Anstelle des Anschlags 53 an dem Innenrohr 50 kann auch ein Bedienrad vorgesehen sein, das den Grad des Einschraubens des Außengewindes 51 des Innenrohrs 50 in dem Innengewinde 32 des Implantats 11 ermöglicht. Je tiefer das Gewinde 51 in das Gewinde 32 hineingeschraubt ist, desto mehr Kraft muss bei der Betätigung des Hebels 82 aufgebracht werden und desto schneller geht die Spannvorrichtung 80 in Selbsthemmung über.

Zur Feinjustierung der Spannung zwischen Innen- und Außenrohr ist am proximalen Ende des Innenrohrs 50 eine Drehscheibe 55 aufgeschraubt.

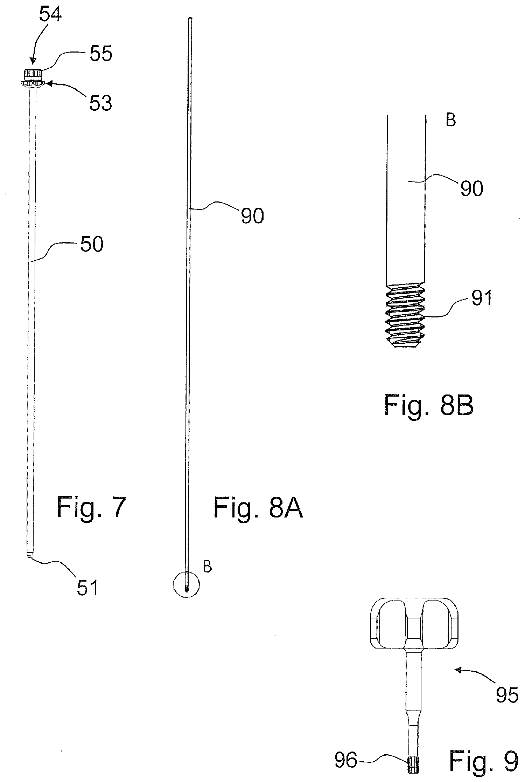

Um das Innenrohr 50 (und auch den Haltestab 90) auf einfache Weise mit dem Implantat 11 zu verschrauben, weist das Innenrohr 50 an seinem proximalen Ende eine Aufnahme 54 für das Mitnehmerprofil 96 eines Schraubendrehers 95 (vgl. Figur 9) auf, mittels dem das Innenrohr 50 einfach mit dem Implantat 11 verschraubt werden kann. Die Verschraubung kann auch erfolgen, wenn in dem Innenrohr 50 zusätzlich der Haltestab 90 eingesteckt ist.

Nachdem die Zahnkränze 24, 24', 64 unsymmetrisch sind, ist es erforderlich, das Implantat 11 in einer bestimmten Richtung an dem Bedieninstrument 60 anzuschrauben. Um die Position des Implantats 11 zu verdeutlichen, ist im Handgriffbereich die Lage des Implantats 11 durch eine entsprechende Anzeige 100 dargestellt.

## Patentansprüche

1. Bedieninstrument (60) zur Implantierung eines Wirbelsäulen-Implantats (11), umfassend ein Außenrohr (61), das an seinem proximalen Ende einen hohlen Handgriff (70) aufweist,
wobei ein Innenstab (50) in dem Außenrohr (61) und dem hohlen Handgriff (70) verschiebbar ist, und der Innenstab (50) mit einem über das distale Ende des Außenrohrs (61) hinausreichenden Gewindebereich (51) versehen ist, wobei der Gewindebereich (51) zur Befestigung am Heckbereich (21) des Implantats (11) dient und zur Verspannung des Implantats (11) mit dessen Heckbereich (21) gegen den distalen Endbereich (67) des Außenrohrs (61) dient, wobei der distale Endbereich (67) des Außenrohrs (61) dem Heckbereich (21) des Implantats (11) zumindest teilweise angepasst ist, **dadurch gekennzeichnet, dass** der Innenstab (50) hohl ist und ein weiterer Haltestab (90) vorgesehen ist, der zur alleinigen Fixierung des Implantats (11) während der Röntgenkontrolle dient, wobei der Haltestab (90) in dem hohlen Innenstab (50) aufnehmbar ist.

2. Bedieninstrument (60) nach Anspruch 1, **dadurch gekennzeichnet, dass** die Verspannung des Implantats (11) gegenüber dem Außenrohr (61) über eine Spannvorrichtung (80) mit einem Hebel (82) erfolgt.

3. Bedieninstrument (60) zur minimal-invasiven oder konventionellen Implantierung eines Wirbelsäulen-Implantats (11) in den Zwischenwirbelraum umfassend ein Außenrohr (61), das an seinem proximalen Ende einen hohlen Handgriff (70) aufweist, wobei ein Innenstab (50) in dem Außenrohr (61) und dem hohlen Handgriff (70) verschiebbar ist, und der Innenstab (50) mit einem über das distale Ende des Außenrohrs (61) hinausreichenden Gewindebereich (51) versehen ist, wobei der Gewindebereich (51) zur Befestigung am Heckbereich (21) des Implantats (11) dient und zur Verspannung des Implantats (11) mit dessen Heckbereich (21) gegen den distalen Endbereich (67) des Außenrohrs (61) dient,
wobei der distale Endbereich (67) des Außenrohrs (61) dem Heckbereich (21) des Implantats (11) zumindest teilweise angepasst ist, wobei die Verspannung des Implantats (11) gegenüber dem Außenrohr (61) über eine Spannvorrichtung (80) mit einem Hebel (82) erfolgt, **dadurch gekennzeichnet, dass** der Hebel (82) einen Exzenter (81) aufweist, der Hebel (82) gelenkig mit einem Rohrstück (88) verbunden ist und bei Betätigung des Hebels (82) das Rohrstück (88) in Längsrichtung verschoben und arretiert wird.

4. Bedieninstrument (60) nach Anspruch 2 oder 3, **dadurch gekennzeichnet, dass** die Spannvorrichtung (80) eine Arretier- und eine Löseposition aufweist und bei Betätigung des Hebels (82) der Spannvorrichtung (80) der Innenstab (50) in dem Außenrohr (61) von einer gelösten Position in eine arretierte Position, in der der Heckbereich (21) des Implantats (11) mit dem distalen Endbereich (67) des Außenrohrs (61) verspannt ist, verschoben wird.

5. Bedieninstrument (60) nach einem der vorangegangenen Ansprüche, **dadurch gekennzeichnet, dass** Zähne eines Zahnkranzes (24, 24') am Heckbereich (21) des Implantats (11) und Zähne eines Zahnkranzes (64) des Bedieninstruments (60) ineinandergreifen.

6. Bedieninstrument (60) nach einem der Ansprüche 2 bis 5, **dadurch gekennzeichnet, dass** der Hebel (82) durch Federelemente (79) in die Löseposition zurückgestellt wird.

7. Bedieninstrument (60) nach einem der Ansprüche 2 bis 6, **dadurch gekennzeichnet, dass** der Exzenter (81) einstückig mit dem Hebel (82) verbunden ist.

8. Bedieninstrument (60) nach einem der Ansprüche 2 bis 7, **dadurch gekennzeichnet, dass** der Exzenter (81) mit einer Unterseite (83) auf einer Gleitfläche (85) des Handgriffs (70) aufliegt.

9. Bedieninstrument (60) nach einem der Ansprüche 2 bis 8, **dadurch gekennzeichnet, dass** die Spannvorrichtung (80) selbsthemmend ist.

10. Bedieninstrument (60) nach einem der Ansprüche 2 bis 9, **dadurch gekennzeichnet, dass** der Hebel (82) an dem Handgriff (70) oder einem gegenüber dem Handgriff (70) verschiebbaren Rohrstück (88) angeordnet ist.

11. Bedieninstrument (60) nach Anspruch 10, **dadurch gekennzeichnet, dass** ein Anschlag (53) am proximalen Ende des Innenstabs (50) auf dem Rohrstück (88) aufliegt und der Innenstab (50) in dem Rohrstück (88) geführt ist und das Rohrstück (88) gegenüber dem Handgriff (70) in Längsrichtung verschiebbar ist.

12. Bedieninstrument (60) nach Anspruch 10 oder 11, **dadurch gekennzeichnet, dass** ein an dem Rohrstück (88) vorgesehenes Federelement (84) den Innenstab (50) in Löseposition außer Eingriff mit dem Heckbereich (21) des Implantats (11) stellt.

13. Bedieninstrument (60) nach einem der Ansprüche 2 bis 12, **dadurch gekennzeichnet, dass** sich im proximalen Endbereich des Handgriffs (70) die Spannvorrichtung (80) zur Verspannung des Innenstabs (50) befindet.

14. Bedieninstrument (60) nach Anspruch 1, 2 oder 4 bis 13, **dadurch gekennzeichnet, dass** der Haltestab (90) an seinem distalen Ende einen Gewindebereich (91) zur Befestigung am Implantat (11) aufweist.

15. Wirbelsäulenimplantat (11) zur Verwendung mit einem Bedieninstrument (60) nach einem der Ansprüche 1, 2 oder 4 bis 14, mit einer Oberseite (12) und einer Unterseite (13), einer vertikalen Durchgangsöffnung (14), einem Vorderende (20) und einem Implantatheck (21), das einen bewegbaren Zapfen (30) mit einem Gewindestück (31) aufweist, **dadurch gekennzeichnet, dass** das Gewindestück (31) ein Innengewinde (32) mit einem größeren Durchmesser zur Befestigung des Innenstabs (50) und ein Gewindestück (33) mit einem kleineren Durchmesser zur Befestigung des Haltestabs (90) aufweist.

## Claims

1. Operating instrument (60) for implanting a spinal implant (11), comprising an outer tube (61) which has a hollow handle (70) on its proximal end, wherein an inner rod (50) can be moved in the outer tube (61) and the hollow handle (70), and the inner rod (50) is provided with a threaded region (51) which extends beyond the distal end of the outer tube (61), wherein the threaded region (51) serves for fastening to the rear region (21) of an implant (11) and for tensioning the implant (11) with its rear region (21) against the distal end region (67) of the outer tube (61), wherein the distal end region (67) of the outer tube (61) is at least partially adapted to the rear region (21) of the implant (11), **characterised in that** the inner rod (50) is hollow and a further holding rod (90) is provided which serves to fix the implant (11) alone during X-ray examination, wherein the holding rod (90) can be received in the hollow inner rod (50).

2. Operating instrument (60) according to claim 1, **characterised in that** the tensioning of the implant (11) against the outer tube (61) takes place by means of a tensioning device (80) with a lever (82).

3. Operating instrument (60) for minimally invasive or conventional implanting of a spinal implant (11) into the intervertebral space, comprising an outer tube (61) which has a hollow handle (70) on its proximal end, wherein an inner rod (50) can be moved in the outer tube (61) and the hollow handle (70), and the inner rod (50) is provided with a threaded region (51) which extends beyond the distal end of the outer tube (61), wherein the threaded region (51) serves for fastening to the rear region (21) of an implant (11) and for tensioning the implant (11) with its rear region (21) against the distal end region (67) of the outer tube (61), wherein the distal end region (67) of the outer tube (61) is at least partially adapted to the rear region (21) of the implant (11), whereby the tensioning of the implant (11) against the outer tube (61) takes place by means of a tensioning device (80) having a lever (82), **characterised in that** the lever (82) having an eccentric (81), the lever (82) is hingedly connected to the tubular piece (88) and, upon actuation of the lever (82), the tubular section (88) is moved in the longitudinal direction and locked.

4. Operating instrument (60) according to claim 2 or 3, **characterised in that** the tensioning device (80) has a locked position and a released position and, upon actuation of the lever (82) of the tensioning device (80), the inner tube (50) in the outer tube (61) is moved from a released position into a locked position, in which the rear region (21) of the implant (11) is tensioned with the distal end region (67) of the outer tube (61).

5. Operating instrument according to one of the preceding claims, **characterised in that** teeth of a sprocket (24, 24') at the rear region (21) of the implant (11) and a sprocket (64) of the operating instrument (60) engage with one another.

6. Operating instrument (60) according to one of the preceding claims 2 to 5, **characterised in that** the lever (82) is put back into the released position by means of spring elements (79).

7. Operating instrument (60) according to one of the preceding claims 2 to 6, **characterised in that** the eccentric (81) being integrally connected to the lever (82).

8. Operating instrument (60) according to one of the proceeding claims 2 to 7, **characterised in that** the eccentric (81) lies with its lower side (83) on a sliding surface (85) of the handle piece (70).

9. Operating instrument (60) according to one of the preceding claims 2 to 8, **characterised in that** the tensioning device (80) is self-locking.

10. Operating instrument (60) according to one of the proceeding claims 2 to 9, **characterised in that** the lever (82) is arranged on the handle (70) or a tubular piece (88) which can be moved with respect to the handle (70).

11. Operating instrument (60) according to claim 10, **characterised in that** a stop (53) lies on the tubular piece (88) on the proximal end of the inner rod (50) and the inner rod (50) is guided in the tubular piece (88) and the tubular piece (88) can be moved in the longitudinal direction with respect to the handle (70).

12. Operating instrument (60) according to claim 10 or 11, **characterised in that** a spring element (84) provided on a tubular piece (88) disengages the inner rod (50) from the rear region (21) of the implant (11) in the released position.

13. Operating instrument (60) according to one of the claims 2 to 12, **characterised in that** the tensioning device (80) is located in the proximal end region of the handle (70) for tensioning the inner rod (50) .

14. Operating instrument (60) according to one of the proceeding claims 1, 2 or 4 to 13, **characterised in that** the holding rod (90) has a threaded region (91) on its distal end for fastening to the implant (11).

15. Spinal implant (11) for use with an operating instrument (60) according to one of the proceeding claim 1, 2 or 4 to 14, having an upper side (12) and a lower side (13), a vertical passage opening (14), a front end (20) and an implant rear (21) which has a moveable cone (30) having a threaded piece (31), **characterised in that** the threaded piece (31) has an internal thread (32) having a larger diameter for fastening the inner rod (50) and a threaded piece (33) having a smaller diameter for fastening the holding rod (90).

## Revendications

1. Instrument de manipulation (60) pour l'implantation d'un implant vertébral (11), comprenant un tube extérieur (61) qui présente une poignée creuse (70) à son extrémité proximale,
une tige intérieure (50) pouvant être déplacée dans le tube extérieur (61) et dans la poignée creuse (70), et la tige intérieure (50) étant pourvue d'une partie filetée (51) qui dépasse de l'extrémité distale du tube extérieur (61), la partie filetée (51) servant à être vissée à la partie arrière (21) de l'implant (11) et à fixer ledit implant (11) en serrant sa partie arrière (21) contre la partie terminale distale (67) du tube extérieur (61), la partie terminale distale (67) du tube extérieur (61) étant adaptée au moins partiellement à la partie arrière (21) de l'implant (11), **caractérisé en ce que** la tige intérieure (50) est creuse et qu'il est prévu une autre tige de maintien (90) qui sert uniquement à la fixation de l'implant (11) pendant le contrôle radiologique, la tige de maintien (90) pouvant être logée dans la tige intérieure creuse (50) .

2. Instrument de manipulation (60) selon la revendication 1, **caractérisé en ce que** la fixation par serrage de l'implant (11) par rapport au tube extérieur (61) est effectuée par l'intermédiaire d'un dispositif de serrage (80) pourvu d'un levier (82) .

3. Instrument de manipulation (60) pour l'implantation mini-invasive ou conventionnelle d'un implant vertébral (11) dans l'espace intervertébral, comprenant un tube extérieur (61) qui présente une poignée creuse (70) à son extrémité proximale, une tige intérieure (50) pouvant être déplacée dans le tube extérieur (61) et dans la poignée creuse (70), et la tige intérieure (50) étant pourvue d'une partie filetée (51) qui dépasse de l'extrémité distale du tube extérieur (61), la partie filetée (51) servant à être vissée à la partie arrière (21) de l'implant (11) et à fixer ledit implant (11) en serrant sa partie arrière (21) contre la partie terminale distale (67) du tube extérieur (61),
la partie terminale distale (67) du tube extérieur (61) étant adaptée au moins partiellement à la partie arrière (21) de l'implant (11), la fixation par serrage de l'implant (11) par rapport au tube extérieur (61) étant effectuée par l'intermédiaire d'un dispositif de serrage (80) pourvu d'un levier (82), **caractérisé en ce que**
le levier (82) présente un excentrique (81) qui est relié à une pièce tubulaire (88) de manière articulée et, lors de l'actionnement du levier (82), la pièce tubulaire (88) est déplacée en sens longitudinal et est arrêtée.

4. Instrument de manipulation (60) selon la revendication 2 ou 3, **caractérisé en ce que** le dispositif de serrage (80) présente une position d'arrêt et une position de libération et, lors de l'actionnement du levier (82) du dispositif de serrage (80), la tige intérieure (50) est déplacée dans le tube extérieur (61) afin de passer d'une position libérée à une position arrêtée dans laquelle la partie arrière (21) de l'implant (11) est fixée par serrage à la partie terminale distale (67) du tube extérieur (61).

5. Instrument de manipulation (60) selon l'une quelconque des revendications précédentes, **caractérisé en ce que** des dents d'une couronne dentée (24, 24') prévues sur la partie arrière (21) de l'implant (11) et des dents d'une couronne dentée (64) de l'instrument de manipulation (60) engrènent les unes dans les autres.

6. Instrument de manipulation (60) selon l'une quelconque des revendications 2 à 5, **caractérisé en ce que** le levier (82) est ramené dans la position de libération par des éléments élastiques (79).

7. Instrument de manipulation (60) selon l'une quelconque des revendications 2 à 6, **caractérisé en ce que** l'excentrique (81) est relié au levier (82) de manière monobloc.

8. Instrument de manipulation (60) selon l'une quelconque des revendications 2 à 7, **caractérisé en ce que** l'excentrique (81) repose avec une face inférieure (83) sur une surface de glissement (85) de la poignée (70).

9. Instrument de manipulation (60) selon l'une quelconque des revendications 2 à 8, **caractérisé en ce que** le dispositif de serrage (80) est autobloquant.

10. Instrument de manipulation (60) selon l'une quelconque des revendications 2 à 9, **caractérisé en ce que** le levier (82) est disposé sur la poignée (70) ou sur une pièce tubulaire (88) qui peut être déplacée par rapport à la poignée (70).

11. Instrument de manipulation (60) selon la revendication 10, **caractérisé en ce qu'**une butée (53) prévue à l'extrémité proximale de la tige intérieure (50) repose sur la pièce tubulaire (88) et que la tige intérieure (50) est guidée dans ladite pièce tubulaire (88) et **en ce que** la pièce tubulaire (88) peut être déplacée en sens longitudinal par rapport à la poignée (70).

12. Instrument de manipulation (60) selon la revendication 10 ou 11, **caractérisé en ce qu'**un élément élastique (84) prévu sur la pièce tubulaire (88) désengage la tige intérieure (50) qui se trouve en position de libération de la partie arrière (21) de l'implant (11).

13. Instrument de manipulation (60) selon l'une quelconque des revendications 2 à 12, **caractérisé en ce que** le dispositif de serrage (80) destiné à fixer la tige intérieure (50) par serrage se trouve dans la partie terminale proximale de la poignée (70) .

14. Instrument de manipulation (60) selon la revendication 1, 2 ou 4 à 13, **caractérisé en ce que** la tige de maintien (90) présente, à son extrémité distale, une partie filetée (91) destinée à être fixée à l'implant (11).

15. Implant vertébral (11) destiné à être utilisé avec un instrument de manipulation (60) selon l'une quelconque des revendications 1, 2 ou 4 à 14, pourvu d'une face supérieure (12) et d'une face inférieure (13), d'un trou de passage vertical (14), d'une extrémité avant (20) et d'une partie arrière d'implant (21) qui présente un tenon mobile (30) pourvu d'une pièce filetée (31), caractérisé que la pièce filetée (31) présente un filetage intérieur (32) ayant un diamètre plus grand pour la fixation de la tige intérieure (50) et une pièce filetée (33) ayant un diamètre plus petit pour la fixation de la tige de maintien (90).
